# EUROPEAN PATENT APPLICATION

(11) **EP 1 792 619 A1**
(43) Date of publication of application: **06.06.2007**
(21) Application number: 05111395.9
(22) Date of filing: 28.11.2005
(51) Int. Cl.: A61K 31/575, A61P 11/00

(54) **Beta-sitosterol for treating porcine respiratory disease complex**

(71) Applicant: LABORATORIOS MAYMO, S.A., 08017 Barcelona (ES)
(72) Inventor:
(74) Representative: Barlocci, Anna

(57) **Abstract**

Use of beta-sitosterol for the prophylactic or therapeutic treatment of pigs susceptible to suffering or suffering from Porcine Respiratory Disease Complex. The veterinarian product is administered in oral form, preferably by feed. The use of beta-sitosterol in the mentioned disease complex is especially advantageous and convenient because this product could be administered for a long period of time without secondary effects.

## Description

The present invention relates to the use of a natural substance for the prophylactic or therapeutic treatment of pigs.

### BACKGROUND ART

Swine production is highly technified and industrialized in a great number of countries. As a consequence of this industrialization, animals are usually confined in reduced space allocations. Overcrowding in farms has generated a big "culture medium" which has been the cause of an increased prevalence of "classic" diseases and of the appearance of new diseases (emergent diseases). This situation has caused that many economically relevant diseases are multifactorial diseases, which means that are the result of a combination of multiple infectious agents, the environment, and various management practices employed by producers.

Clinical problems caused by multifactorial diseases appear frequently during the swine production phase that takes place from the three or four weeks (weaners) until twelve or thirteen weeks (growers) of age. These clinical problems lead to an increase of mortality and to a worsening of zootechnical parameters, which significantly increase the final production cost of pigs. These pathological problems have become worse as a result of the intensification of swine production, and mainly as a consequence of the existence of viral and mycoplasmal diseases, such as Porcine Reproductive and Respiratory Syndrome (PRRS), Postweaning Multisystemic Wasting Syndrome (PMWS), and Mycoplasma hyopneumoniae infection.

One of the most common conditions responsible for grow-finish inefficiency in swine farms is Porcine Respiratory Disease Complex (PRDC). This complex is most often due to the interaction of multifactorial etiologies, being the result of a combination of multiple infectious agents, environmental stressors and production system challenges. It can affect pigs from birth through finishing. Its clinical consequences and its economic impact could be usually observed throughout the finishing period. Successful intervention requires accurate diagnosis, properly time vaccinations, strategic use of medication, eliminating environmental stressors, and in some cases, changes in production methods.

Both viral and bacterial organisms play a role, as does the environment, and various management practices employed by producers. When in the right combination, these factors can sufficiently compromise the pigs' respiratory defense mechanisms, resulting in severe respiratory disease. Common pathogens isolated from PRDC cases include Mycoplasma hyopneumoniae, PRRS virus, porcine circovirus type 2 (PCV2), swine Influenza virus, Pasteurella multocida, and other bacteria such as Actinobacillus pleuropneumoniae, Salmonella cholerasuis, Hemophilus parasuis and Streptococcus suis. Among those pathogens, especially PRRS virus, PCV2 (etiologic agent of PMWS), and Mycoplasma hyopneumoniae produce chronic diseases.

The swine respiratory pathologies cause a variable mortality and morbidity, but always involve a delay in the growth of the animals, a worst conversion index, and the appearance of culls. PRDC can be considered a very important economical problem to swine producers all over the world. Presently, there are several control strategies and/or therapeutic measures to reduce the clinical problems.

One control strategy is the early weaning which consists in removing suckling piglets from their mother at about 3 weeks of age to prevent their infection. Piglets are allocated in spaces without having contact with their mothers or with other pigs of different ages. However, this measure has resulted not effective against many microorganisms, as transmission from the mother could take place through "in-utero" transmission or throughout the first days of life.

Another control strategy is the use of vaccines both in mothers and progeny Vaccination has several drawbacks when is used as a control strategy for multifactorial diseases, firstly because effective vaccines for all microorganisms which may be involved in pathological processes are not available, and secondly because other factors such as environment, density of population, etc. are also involved. Moreover, microorganism strain present in the vaccine and their immunogenicity are some other factors that could diminish their effectiveness under field conditions.

Another strategy is the administration of antibiotic substances through feed and/or water to the whole pig population, when the disease is detected, with the aim of diminish morbidity and mortality (prophylactic treatment). Aside of the high cost of this treatment, the main drawback is that also healthy animals are treated. Nowadays, there is a trend to limit antibiotic use to reduce antimicrobial resistance both in animal and in human population.

Finally, another strategy is the use of more resistant breeds, strains or genetic lines in front of the involved diseases. This has several drawbacks. The first one is the scarcity of knowledge available in this matter and the high cost of implementation when this strategy is applied for the whole population.

In summary, although several strategies are used to reduce the clinical signs, lesions and mortality of pigs caused by Porcine Respiratory Disease Complex, a totally satisfactory treatment has yet to be found. Furthermore, they do not fully address the real need of breaking the cycle of infection. As a result, active research has continued in this field.

### SUMMARY OF THE INVENTION

Inventors have found that administering beta-sitosterol to pigs affected by porcine respiratory disease complex, a surprisingly improvement of the general health status of the animals, a significant improvement of zootechnical parameters, and the reduction of clinical signs, lesions and mortality of pigs are observed. Thus, a more effective swine production is achieved. Moreover, administration of beta-sitosterol allows the reduction of use of antibiotics for prophylactic or therapeutic purposes, in case a combined therapy with antibiotics is also used.

Beta-sitosterol (BSS) and its glycoside (BSSG) are sterol molecules are synthesized by plants. When humans eat plant foods phytosterols are ingested, and are found in the serum and tissues of healthy individuals, but at concentrations orders of magnitude lower than endogenous cholesterol. In animals, Beta-sitosterol (BSS) and its glycoside (BSSG) have been shown to exhibit anti-inflammatory, anti-neoplasic, anti-pyretic and immune-modulating activity (cf. P.J.D. Bouic et al., Int. J. Immunopharmacol. 1996, vol. 18(2), pp. 693-700). EP 509.656 discloses the use of a combination of BBS and BSSG in the treatment of a disease involving perturbations of normal lymphocyte homeostasis and functionality in humans. Some studies about the effect of BSS:BSSG mixture in the treatment of human immunodeficiency virus (HIV) and in feline immunodeficiency virus (FIV) (cf. Lamprecht et al, Int Conf AIDS 1998 Jun 28-Jul 3; 12:29 (abstract no. 11236); U. Breytenbach et al, Cell Biology international., 2001, vol. 25(1), pp. 43-49). WO 01/00194 discloses a composition comprising inositol hexaphosphate and a 50:50 mixture of beta-sitosterol and beta-sitosterolin in the treatment of chronic immune conditions, such as cancer, AIDS/HIV, hepatitis-C, herpes, colds, influenza, bronchitis, chronic fatigue and Epstein Barr, in a mammal.

Nevertheless, in the prior art no mention is made about the use of beta-sitosterol for the treatment of pigs susceptible to suffering or suffering from swine enzootic diseases, such as Porcine Respiratory Disease Complex. Neither a mention is made about the use of beta-sitosterol for the general treatment of swine nor is any teaching that could point to this use disclosed.

Although, BSS:BSSG mixture has shown efficacy in controlling the FIV in cats, it was impossible to foresee from the experience obtained in cats that the treatment with beta-sitosterol would also work in other species, such as pigs, to control a disease caused by different infectious agents, such as PRDC. Additionally, PRDC is most often due to the interaction of multifactorial etiologies, being the result of a combination of multiple infectious agents, environmental stressors, and production system challenges.

Accordingly, an aspect of the invention relates to the use of beta-sitosterol for the preparation of a veterinarian product for the prophylactic or therapeutic treatment of pigs susceptible to suffering or suffering from Porcine Respiratory Disease Complex.

The invention also relates to a method for prophylactic or therapeutic treatment of pigs susceptible to suffering or suffering from Porcine Respiratory Disease Complex. This method comprises administering to pigs a veterinarian product comprising a prophylactically or therapeutically effective amount of beta-sitosterol.

The term "swine enzootic disease" is used to describe any pig disease which is constantly present in a swine population.

The term "field conditions" relates to the normal conditions (facilities, nutrition, management) which take place in a standard (normal) swine production system.

The term "historical control" relates to the zootechnical or clinical results collected during a long period of time from a production system where there is adequately documented information about a disease or condition, or with a regimen (therapeutic, diagnostic, prophylactic) whose effectiveness is established. This information will be quantitatively compared with the results of the new treatment.

According to Royal Decree 56/2002, the term "complementary feed" relates to mixtures of foodstuffs comprising high percentages of specific substances, and that, according to its composition, only guarantees the daily portion if in association with other foodstuffs for animals.

### DETAILED DESCRIPTION OF THE INVENTION

As mentioned above, according to one aspect of the invention, beta-sitosterol is used for the preparation of a veterinarian product for the treatment of pigs susceptible to suffering or suffering from PRDC. Among the pathogens usually present in PRDC, porcine reproductive and respiratory syndrome virus, porcine circovirus type 2, and Mycoplasma hyopneumoniae produce chronic diseases. Accordingly, in a preferred embodiment at least one of the pathogens causing the disease complex is selected from porcine reproductive and respiratory syndrome virus, porcine circovirus type 2, and Mycoplasma hyopneumoniae. When one of these three pathogens is involved in the mentioned disease complex, the use of beta-sitosterol is especially advantageous and convenient because this product could be administered for a long period of time without secondary effects.

Notwithstanding the treatment with beta-sitosterol, when necessary, drugs (parenteral antibiotics, antibiotics by water or feed) may be used in those cases where a bacterial infection is suspected (according to the clinical criteria of the veterinarian) in order to control the disease. The kind of antibiotic is unable to be determined previously. The antibiotics that could be potentially used must fulfill the criteria of selection according to Government regulations on antibiotics used in farm animals. The use of antibiotics is compatible with the supplementation with beta-sitosterol, since this one does not intend to substitute the effect of antibiotics commonly used for prophylactic or therapeutic purposes. In any case, the amount of antibiotic will be reduced, which is an advantage derived from the use of beta-sitosterol, mainly in infections caused by pathogens which produce a long time clinical course.

Beta-sitosterol is preferably administered in oral form. It may be administered as a feed additive or otherwise consumed by pigs. Preferably, administration of beta-sitosterol is carried out by feed or via water supply. The amount of beta-sitosterol orally administered is incorporated in a portion of the total dietary intake of feed or water. The lower dose will be determined by the minimum amount having the expected effect, being the higher dose dependent on the economic viability of the treatment, as there is no limit from the toxicological point of view. When administered by feed, preferably, the effective amount of beta-sitosterol is between about 20 to about 200 g beta-sitosterol/1,000 kg of feed, more preferably between about 110 to about 130 g beta-sitosterol/1,000 kg of feed. Beta-sitosterol (active ingredient) may be administered through a complementary feed mixed with other ingredients commonly used in swine feeds. Roasted soybean flour and palm oil may be mentioned as an example of some of the ingredients that may be present in the complementary feed comprising beta-sitosterol. Accordingly, in a preferred embodiment the veterinarian product comprising beta-sitosterol is a complementary feed. The complementary feed can be designed to meet the final daily intake of the active ingredient and varied according to this criteria. Preferably, the complementary feed that is used to administer the active ingredient comprises an amount of beta-sitosterol between about 10 to about 100 g beta-sitosterol/kg, more preferably between about 55 to about 65 g beta-sitosterol/kg. Although the period of administration must be decided case by case in order to adapt the treatment with the clinical expression of the disease in each farm, preferably, beta-sitosterol is administered throughout the period from 5 to 13 weeks of life.

Oral administration of beta-sitosterol allows treating a big population of pigs in an easy and effective way without the risk of secondary effects in healthy pigs. Another advantage of the treatment with a diet supplemented with beta-sitosterol is the significant decrease in production costs.

Throughout the description and claims the word "comprise" and variations of the word, such as "comprising", is not intended to exclude other technical features, additives, components, or steps. Additional objects, advantages and features of the invention will become apparent to those skilled in the art upon examination of the description or may be learned by practice of the invention. The following examples and drawings are provided by way of illustration, and is not intended to be limiting of the present invention.

### EXAMPLES

### Example 1: Effect of the administration of beta-sitosterol in pigs farms suffering from Porcine Respiratory Disease Complex.

The objective of this study was to evaluate the potential benefit of supplementing pigs with beta-sitosterol. To assess the efficacy of the administration of beta-sitosterol, clinical and zootechnical results considered standard in several production systems (historical controls) were compared with the results obtained after applying the experimental treatment. The treatment was the only factor changed in the production system.

Data of the experimental treatment were obtained administering the product under field conditions in 40 swine farms in Spain during more than 1 year. Finally, clinical and zootechnical data before (historical control, data from 20,000 pigs) and after administration of beta-sitosterol (data from 10,000 pigs) were compared. Due to the huge amount of animals involved in the comparison study and being the treatment the only factor changed in the production system, the results obtained are statistically significant.

### Materials and methods

### Animals, housing and diet

The study was carried out with pigs from nursery (weaners) and/or finisher phase (growers). The pigs were housed in pens containing between 10 and 25 animals depending on their phase of production (growers vs. weaners).

The space available for the animals was adapted to the standard needs for each category of animal, which are 0.2-0.25 m² and 0.65-0.80 m² for the nursery and finisher phases, respectively. This density is considered adequate under commercial conditions.

The buildings were equipped with manual or automatic mechanisms for controlling ventilation.

The animals were fed and watered ad libitum. The feed, stored at ambient temperature, was distributed in hoppers (one per pen) and the water was supplied through an automated system. The administration of beta-sitosterol to treated pigs was carried out by feed with the standard diet plus beta-sitosterol. Beta-sitosterol was administered mixed with feed at a dosage of 120 ppm, i.e. 120 g of beta-sitosterol per 1,000 kg of feed. The feed met the nutritional requirements for each age group according to the National Research Council (NRC) tables. The product was administered from the 5 to the 13 weeks of life of pigs. The period of administration must be decided case by case in order to adapt the treatment with the clinical expression of the disease in each farm. For convenience, beta-sitosterol was administered trough a complementary feed comprising an 88.5% of roasted soybean flour, a 3.0% of palm oil, and an 8.5% of a commercially available mixture of pine steroids comprising more than a 70% of beta-sitosterol. The amount of beta-sitosterol comprised in the complementary feed was of about 60 g/kg. The complementary feed was administered at a rate of 2 kg per 1,000 kg of complete feed during the 8 weeks of the study. Taking into account the feed intake, from 4 to 6 mg/Kg body weight/day of beta-sitosterol were administered to pigs from 40 to 20 kg of body weight, respectively.

When necessary, antibiotics were used in those cases where a bacterial infection was suspected in order to control the disease. Two vaccinations with Aujeszky's disease virus modified live vaccine were applied at 10 and 13 weeks of life according to Spanish mandatory legislation.

Collection of data

All the piglets were followed up daily until the end of the trial period by the responsible veterinarian of the farm.

Criteria of efficacy of beta-sitosterol include parameters of average daily gain, number of culls and mortality. Thus, for the comparison between untreated and treated groups, those parameters were taken into account. Average daily gain (ADG) was calculated as the difference between the final and the initial weight divided by the number of days of the study.

### Results

Table 1 shows the percentage of mortality observed in pigs before (Group A; data of 20,000 animals) and after (Group B; data of 10,000 animals) the administration of the beta-sitosterol, according to the phase of production.

**Table 1**

| Phase of production | Group A | Group B |
|---|---|---|
| weaners (3-8 weeks of life) | 5-6 | 1.5-2.5 |
| growers (8-24 weeks of life) | 12-15 | 7-8 |
| Total mortality | 17-21 | 8.5-10.5 |

Table 2 shows the percentage of culls (pigs not suitable for commercialization) remaining in the swine farms after finishing their normal productive phase before (Group A; data of 20,000 animals) and after (Group B; data of 10,000 animals) the administration of the beta-sitosterol, according to the phase of production.

**Table 2**

| Phase of production | Group A | Group B |
|---|---|---|
| weaners (3-8 weeks of life) | 2-3 | 0.1-0.5 |
| growers (8-24 weeks of life) | 3-4 | 0,5-1 |
| Total culls | 5-7 | 0.6-1.5 |

Additionally, Table 3 shows the substantially improvement of zootechnical parameters during the grower phase. The ADG was calculated as the difference between the final and the initial weight divided by 56 (length of the trial).

**Table 3**

| Parameter | Group A | Group B |
|---|---|---|
| Days to gain weight | 135 | 123 |
| Technical conversion | 2.798 | 2.571 |
| Economical conversion | 3.002 | 2.692 |
| Average daily gain (ADG) | 572 | 668 |

The results of the present study show that administering beta-sitosterol by feed to swine the general health status of the animals is improved. More specifically, a significant improvement of zootechnical parameters and the reduction of clinical signs, lesions and mortality caused by Porcine Respiratory Disease Complex in treated versus untreated pigs are observed.

As can be seen from the results of the study, a standard diet supplemented with beta-sitosterol implies a reduction of total mortality close to 50%. It was also observed a reduction close to 79% of culls in swine farms with animals suffering from Porcine Respiratory Disease Complex. Zootechnical parameters during the grower phase have also been substantially improved, as well as corporal condition, in animals that feed this meal from 5 to 13 weeks of life in comparison with pigs that receive a standard diet during the same period of time.

## Claims

1. Use of beta-sitosterol for the preparation of a veterinarian product for the prophylactic or therapeutic treatment of pigs susceptible to suffering from Porcine Respiratory Disease Complex.

2. Use according to claim 1, wherein at least one of the pathogens causing the disease complex is selected from porcine reproductive and respiratory syndrome virus, porcine circovirus type 2, and Mycoplasma hyopneumoniae.

3. Use according to any one of claims 1 to 2, wherein the veterinarian product is administered in oral form.

4. Use according to claim 3, wherein said administration is carried out by feed.

5. Use according to claim 4, wherein the administered amount of beta-sitosterol is comprised between about 20 to about 200 g beta-sitosterol/1.000 kg of feed.

6. Use according to any one of claims 1 to 5, wherein the veterinarian product is a complementary feed.

7. Use according to claim 6, wherein the complementary feed comprises an amount of beta-sitosterol between about 10 to about 100 g beta-sitosterol/kg.

8. Use according to claim 3, wherein said administration is carried out with water supply.

9. Use according to any one of claims 1 to 8, wherein the veterinarian product is administered throughout the period from 5 to 13 weeks of life.
